# EUROPEAN PATENT SPECIFICATION

(11) **EP 4 393 522 B1**
(45) Date of publication and mention of the grant of the patent: **11.11.2026**
(21) Application number: 22460080.9
(22) Date of filing: 29.12.2022
(51) Int. Cl.: A61L 27/36

(54) **IMPLANT FOR THE RECONSTRUCTION OF THE URINARY BLADDER, THE METHOD OF ITS PREPARATION AND THE SCAFFOLD FOR THE IMPLANT FOR THE RECONSTRUCTION OF THE URINARY BLADDER**
IMPLANTAT ZUR REKONSTRUKTION DER HARNBLASE, VERFAHREN ZU SEINER HERSTELLUNG UND GERÜST FÜR DAS IMPLANTAT ZUR REKONSTRUKTION DER HARNBLASE.
IMPLANT POUR LA RECONSTRUCTION DE LA VESSIE URINAIRE, SON PROCÉDÉ DE PRÉPARATION ET ÉCHAFAUDAGE POUR L'IMPLANT POUR LA RECONSTRUCTION DE LA VESSIE URINAIRE.

(43) Date of publication of application: 03.07.2024
(73) Proprietor: Uniwersytet Mikolaja Kopernika w Toruniu, 87-100 Torun (PL)
(72) Inventor: Pokrywczynska, Marta, 88-180 Zlotniki Kujawskie (PL); Adamowicz, Jan, 85-065 Bydgoszcz (PL); Kloskowski, Tomasz, 85-796 Bydgoszcz (PL); Balcerczyk, Daria, 11-740 Piecki (PL); Rasmus, Marta, 85-796 Bydgoszcz (PL); Szulc, Marta, 82-300 Elblag (PL); Fekner, Zuzanna, 85-301 Bydgoszcz (PL); Stopel, Michal, 85-794 Bydgoszcz (PL); Jundzill, Arkadiusz, 85-363 Bydgoszcz (PL)

(56) References cited:
- PENG XU ET AL: "Development and characterization of bladder acellular matrix cross-linked by dialdehyde carboxymethyl cellulose for bladder tissue engineering - RSC Advances (RSC Publishing) DOI:10.1039/C9RA07965C", RSC ADV., 18 December 2019 (2019-12-18), XP093076631, Retrieved from the Internet <URL:https://pubs.rsc.org/en/content/articlehtml/2019/ra/c9ra07965c> [retrieved on 20230828]
- BAM PEMO ET AL: "Design of biostable scaffold based on collagen crosslinked by dialdehyde chitosan with presence of gallic acid", INTERNATIONAL JOURNAL OF BIOLOGICAL MACROMOLECULES, vol. 130, 4 March 2019 (2019-03-04), pages 836 - 844, XP085660026, ISSN: 0141-8130, DOI: 10.1016/J.IJBIOMAC.2019.03.017

## Description

The subject of the invention is an graft for the reconstruction of the urinary bladder, a method of its preparation and a scaffold for the implant for the reconstruction of the urinary bladder, built using tissue engineering techniques, used in reconstructive urology, especially in the reconstruction of the human bladder wall, stimulating the regeneration of tissues of the urinary system.

The most common indication for reconstruction of the bladder wall (cystoplasty) is the so-called small highpressure bladder, resulting from congenital and developmental defects of the urinary tract, chronic inflammation, neurogenic dysfunction, radiation damage, injuries and other diseases. The purpose of cystoplasty is to increase the capacity of the reconstructed bladder, reduce intravesical pressure, and prevent urine from flowing back to the kidneys and damaging them.

The currently used techniques of bladder reconstruction using the wall of the gastrointestinal tract carry the risk of many complications resulting from the additional surgical procedure on the intestine and the different properties of the wall of the gastrointestinal tract and the urinary tract. The result is electrolyte and osmotic imbalance, neurological disorders, bone demineralization, infections, stone formation and an increased risk of carcinogenesis at the anastomotic sites of the urinary tract epithelium.

At the moment, there is no implant for the reconstruction of the urinary tract to replace the currently used autologous material - the wall of the patient's gastrointestinal tract. Currently, various biomaterials are used in experimental research on the regeneration of tissues and organs using tissue engineering techniques. A special role is played by biomaterials obtained by decellularization, i.e. removal of cells from tissues or organs of animal organisms. The decellularization process consists in the complete removal of cells and their remains (nucleic acids) from tissues or entire organs using physical, chemical and biological methods. The matrix obtained in this way is made of structural proteins of the extracellular matrix, mainly collagen and elastin.

The best-known commercially available products used in clinical practice, based on cell-free matrices, are:
- SIS (Small Intestinal Submucosa), a cell-free scaffold made from the submucosa of the porcine small intestine wall, commercially available as Surgisis^{®} from Cook Medical. This material is used in the treatment of hernias, perianal fistulas, dural plastic surgery, otological procedures (myringoplasty, tympanoplasty) and gastroenterological procedures (rectal prolapse surgery);
- decellularized bovine or equine pericardium, commercially available under the name Veritas^{®} or OrthAdapt^{®} from Synovis Life Tech.; this material is used in cardiac surgery in the repair of cardiovascular defects;
- decellularized human or porcine skin, commercially available as AlloDerm^{®} or Strattice^{™} from LifeCell Corp. as a material used as a skin substitute in the treatment of burns and breast reconstruction procedures;
- Bladder Acellular Matrix (BAM) is commercially available as Matristem UBM and is intended for the treatment of difficult-to-heal wounds.

BAM matrices produced from the bladder wall are used in experimental research on bladder reconstruction. The main problems associated with the use of cell-free BAM matrices in bladder reconstruction are: insufficient biocompatibility, urinary permeability and implant fibrosis. Peng ET AL (RSC Advances, 18 December 2019, v.9, p.42000-42009) discloses an example of a bladder acellular matrix (BAM) cross-linked with oxidized carbomethyl cellulose for bladder reconstruction.

The main problem of the present invention is to ensure the best possible biocompatibility of the implant with the patient's tissues, to prevent leakage of urine and to regenerate the components of the bladder wall with the limitation of the area of fibrosis in the central part of the implant.

The aim of the invention is to develop such an implant for the reconstruction of the human bladder, a method of its preparation and a scaffold for the implant for the reconstruction of the urinary bladder, which will eliminate the need to use gastrointestinal tissues in reconstructive surgeries of the urinary tract. The implant according to the invention is characterized by biological and mechanical properties similar to the native bladder wall, it is impermeable to urine and fully biocompatible.

The aim of the invention is to develop such an implant for the reconstruction of the human bladder, a method of its preparation and a scaffold for the implant for the reconstruction of the urinary bladder, which will eliminate the need to use gastrointestinal tissues in reconstructive surgeries of the urinary tract. The implant according to the invention is characterized by biological and mechanical properties similar to the native bladder wall, it is impermeable to urine and fully biocompatible.

The advantage of the invention, compared to known solutions, is that there is no need to perform an additional surgical procedure on the patient's intestine, and thus shortening the total time of the operation and the lack of related complications. The implant is a product available "straight from the shelf".

The implant is characterized by technical parameters enabling its use in laparoscopic surgery. Shape memory enables insertion of the implant through a laparoscopic or robotic trocar. The shape of the implant, configured using computer modeling, minimizes the area of unfavorable environment for regeneration, allowing for even revascularization and colonization of the implant by urothelial and smooth muscle cells. The shape of the implant allows it to be implanted in the bladder wall without the need to create additional cavities. The shape of the implant was designed so that the urine storage pressure in the reconstructed bladder was between 20-60 ml/ cm H₂O. By sealing the BAM matrix with polymers, the resulting composite scaffold is impermeable to urine.

The unique feature of the invention is the growth of cells not only on the surface of the implant, but also inside its three-dimensional structure.

The essence of invention is implant for the reconstruction of the urinary bladder, characterized in that it is in the form of a multi-arm, preferably three-arm, axisymmetric element with arms inscribed in a circle and made of acellular matrix cross-linked with a polymer, the acellular matrix being made of decellularized tissue or an organ of a mammal, preferably human or pig most preferably from the bladder or intestinal wall or skin or dura or pericardium or fragments thereof. Preferably it has a shape with arms resembling a lily petal inscribed in a circle with an area of 3 cm2 to 60 cm2, preferably 30 cm2, described inside a diameter D of 2.9 cm to 14 cm, preferably 9, 45 cm and a thickness of 0.1 cm to 1 cm, preferably 0.5 cm, with an angle of opening alpha from 8° to 37°, preferably 26°, radius R1 from 4.5 mm to 20.5 mm, preferably 14.3 mm and radius R2 from 2.2 mm to 10.02 mm preferably 7.08 mm, and apex angle beta from 24° to 110° preferably 77.7°, preferably the graft size of any surface (S) for D, R1 and R2 multiplies times the scale factor (x) based on the formula: $x = \sqrt{\frac{S}{30}}$

Preferably the polymer is type I or II or III or IV collagen, preferably of animal origin, or cellulose or collagen with cellulose in a ratio of 0.1:2, preferably 1:1, or gelatin or starch or natural silk or cellulose esters and ethers preferably carboxymethyl cellulose or carboxyethyl cellulose or methyl cellulose or chitosan esters and ethers preferably N or O-carboxymethyl chitosan or O-methyl carboxy chitosan or hyaluronic acid or fibrinogen or a synthetic polymer soluble in organic solvents preferably poly(lactide) or poly (glycolide) or poly(caprolactone) or polyamide or polyimide or poly(trimethylene carbonate) or poly(dioxanone) or poly(vinyl acetate) or poly(vinyl alcohol) or poly(N-vinyl-2-pyrrolidone) or polyurethane or poly (etherurethane) or polycarbonate or aromatic polycarbonate or polyethylene oxide or polyethylene glycol or polyacrylic acid or polyacrylamide or polyacrylonitrile or poly(vinyl chloride) or a mixture thereof in a ratio of 0.1:2 to 2:1 or a copolymer of preferably L-lactide and caprolactone or poly(L-lactide-co-caprolactone). Preferably the polymer-crosslinked cell-free matrix comprises on the surface or inside mesenchymal stromal cells isolated from bone marrow, subcutaneous fat, Wharton's jelly or other vascularized human tissues, preferably in a density of 1x10⁶ to 100x10⁶ preferably 10x10⁶ cells/cm².

The essence of invention is a method of obtaining an implant for the reconstruction of the bladder, the obtained tissue or organ of a mammal, preferably human or porcine, preferably from the bladder or intestinal wall or skin or dura or pericardium or fragments thereof, is rinsed with a washing solution and subjected to decellularization, and the cell-free matrix thus obtained is sealed by dipping, spraying, padding or coating with a polymer with a polymer mass concentration of 0.5 to 2%, preferably 1% in hydrochloric acid with a molar concentration of 0.01M to 0.05M, preferably 0.17M and dried, and then the BAM composite thus obtained is crosslinked by dipping, spraying, padding or coating with a crosslinking solution, preferably a dialdehyde natural polymer with a mass concentration of 0.1 to 5%, preferably 1% and in PBS from 10x to 1x, preferably 1x , heats, puts in a Petri dish and adds PBS solution until the BAM composite is completely covered and incubated, then it is re-frozen, dried and sterilized with ethylene oxide, and the thus obtained matrix is punched to a given shape, where the polymer is type I or II or III or IV collagen, preferably of animal origin, or cellulose or collagen to cellulose in a ratio of 0.1:2, preferably 1:1, or gelatin or starch or natural silk or cellulose esters and ethers preferably carboxymethylcellulose or carboxyethylcellulose or methylcellulose or chitosan esters and ethers preferably N or O-carboxymethylchitosan or O-methylcarboxychitosan or hyaluronic acid or fibrinogen or a synthetic polymer soluble in organic solvents preferably poly(lactide) or poly(glycolide) or poly(caprolactone) or polyamide or polyimide or poly(trimethylene carbonate) or poly(dioxanone) or poly(vinyl acetate) or poly(vinyl alcohol) or poly(N-vinyl-2-pyrrolidone) or polyurethane or poly(etherurethane) or polycarbonate or aromatic polycarbonate or poly(ethylene oxide) or poly(ethylene glycol) or poly(acrylic acid) or poly(acrylamide) or poly(acrylonitrile) or poly(vinyl chloride) or a mixture thereof in a ratio of 0.1:2 to 2:1 or a copolymer of preferably L-lactide and caprolactone or poly(L-lactide-co-caprolactone), and after heating, a solution of sodium borohydride, preferably 0.1 to 2%, most preferably 1%, dissolved in ethanol with a concentration of 90 to 99%, preferably 96%, is applied to the surface of the BAM composite , and allowed to stabilize, preferably for 1 to 5 hours, most preferably for 3 hours, preferably at a temperature of 0 to 10°C, most preferably 4°C, preferably a 0.1 M glycine solution is applied to the surface of the BAM composite and then rinsed with a PBS solution, and before or after cutting a predetermined shape onto a matrix from the outer part, previously cultured stromal mesenchymal cells isolated from bone marrow, subcutaneous fat, Wharton's jelly or other vascularized human tissues are seeded in density from 1x10⁶ to 100x10⁶, preferably 10 x 10⁶ cells/cm² by puncturing the implant preferably to a depth of from 0.1 mm to 5 mm, preferably to 2 mm, and injecting the cells, or by depositing the cells on the surface of the implant, and then placing the matrix in the bioreactor and incubates, most preferably for 1 to 30 days, preferably 7 days, in an atmosphere of 2 to 10%, preferably 5% CO₂, and a temperature of 22 to 40°C, preferably 37°C characterized in that the matrix has a shape in the form of a multi-arm, preferably three-arm, axisymmetric element with arms inscribed in a circle and made of a cell-free matrix cross-linked with a polymer, the cell-free matrix being made of from a decellularized tissue or organ of a mammal, preferably human or porcine, most preferably from the bladder or intestinal wall or skin or dura or pericardium or fragments thereof. Preferably the scaffold has a shape with arms resembling a lily petal inscribed in a circle with an area of 3 cm2 to 60 cm2, preferably 30 cm2, described inside a diameter D of 2.9 cm to 14 cm, preferably 9.45 cm, and a thickness of 0.1 cm to 1 cm, preferably 0.5 cm, with a flare angle alpha from 8° to 37°, preferably 26°, radius R1 from 4.5 mm to 20 .5 mm, preferably 14.3 mm, and the radius R2 from 2.2 mm to 10.02 mm, preferably 7.08 mm, and the apex angle beta from 24° to 110°, preferably 77.7°, preferably when the size of the implant of any surface ( S) for D, R1 and R2, multiply the scale factor (x) by the formula: $x = \sqrt{\frac{S}{30}}$

The construction of the implant for bladder augmentation is a composite made of: acellular matrix obtained by decellularization technique from mammalian tissues/organs and polymers.

The acellular matrix is formed by decellularization of a mammalian urinary bladder (Bladder Acellular Matrix, BAM), preferably a porcine or human bladder. Acellular matrix can also be formed as a result of decellularization of other tissues such as the intestinal wall, skin, dura mater, pericardium and others.

The BAM matrix is sealed with a polymer and cross-linked with a natural dialdehyde polymer, preferably resorbable dialdehyde cellulose or dialdehyde starch, dialdehyde chitosan, dialdehyde hyaluronate. The degree of oxidation of the natural dialdehyde polymer is from 20 to 90%, preferably 80%, with an acidic pH, preferably about 3, where the application of the polymer layer relative to the area [cm2] of the BAM matrix ranged from 0.01 to 0.05 g per cm² , preferably 0.02g/cm². Other cross-linking agents may be: glutaraldehyde or other aliphatic and aromatic diamine compounds, genipine, EDC/NHS, citric acid, sodium salt of citric acid, tannic acid and other polyphenols, pentasodium triphosphate, polyphenolic extracts (e.g. wine extract, bitter orange peel, cranberry extract, green tea extract). It should be noted that any suitable polymer or agent that will contribute to the sealing and/or physico-chemical properties of the cell-free matrix will be suitable for use in the method of the present invention.

The surface of the implant may also be enriched/saturated with growth factors such as platelet-derived growth factor (PDGF-BB), fibroblast growth factor (bFGF), vascular endothelial growth factor (VEGF), hepatocyte growth factor (HGF), epidermal growth factor (EGF), transforming growth factor-β (TGF-β) and bone morphogenetic protein 2 (BMP2).

The outer surface of the bladder augmentation implant may be additionally coated with autologous or allogeneic mesenchymal stromal/stem cells isolated from bone marrow, subcutaneous fat, Wharton's jelly or other vascularized tissues. The implant can be covered with cells using any technique - e.g. by applying a cell suspension of the assumed density to the surface of the implant and leaving it for 15 minutes to an hour in an incubator at 37°C in an atmosphere of 5% CO₂ in order to attach the cells to the scaffold or by injection with a needle and syringe. The cells are seeded at a density of 1-100 x 10⁶ cells/cm², preferably 10 x 10⁶ cells/cm², and the cell culture on the scaffold can be carried out under static or dynamic conditions, preferably using a bioreactor, for a period of 1-30 days, preferably 7 days.

The method of producing a BAM matrix for bladder augmentation consists in the fact that the entire bladder or tissue or organ of a mammal, preferably human or pig, most preferably from the bladder or intestinal wall or skin or dura mater or pericardium or fragments thereof, is subjected to dynamic decellularization, during which the decellularizing solution is instilled into/out of the bladder, or static decellularization, where the bladder or a fragment thereof is immersed in the decellularizing solution.

Decellularization of the bladder can be performed by all known methods leading to the removal of cells and their residues from the extracellular matrix of the bladder while maintaining its proper ultrastructure. Prior to decellularization, the urinary bladder or its fragment is rinsed with a washing solution, e.g. distilled water, saline, culture medium, preferably PBS with EDTA, in order to flush out the residual urine. Then, for initial cell lysis, the bladder is frozen and thawed three times, respectively at -1°C to -196°C, preferably -80°C and at 1°C to 40°C, preferably 37°C. In the next stage, the bladder is placed in a vessel filled with an enzymatic solution, additionally, in the case of dececlularization of the entire bladder, the enzyme solution is also instilled into the bladder. It is preferred to use trypsin at a concentration of 0.01% to 50%, preferably 0.25%, for 30 minutes to 48 hours, preferably 2 hours, with a flow rate of 1 liter/hour to 700 liters/hour, preferably 280 liters/hour.

The bladder is then placed and rinsed in a hypotonic Tris buffer solution with a mildly alkaline pH of 8 to 11, which solution may contain active amounts of a proteolytic agent inhibitor e.g. EDTA, or a hypertonic Tris buffer solution which contains a detergent e.g. Triton X-100 or/and SDS or/and SD and/or 3-[(3-cholamidopropyl)-dimethylammonio]-1-propanesulfonate (CHAPS), preferably at a concentration of 0.01% to 20%, which may contain active amounts of proteolytic inhibitors, preferably EDTA in an amount of 0.01 mM to 20 mM, preferably 5 mM for 12 to 96 hours, preferably 72 hours for Triton X-100 and 24 hours for SDS, with a flow rate of 1 liter/hour to 700 liters/hour preferably 280 liters/hour.

The hypotonic solution or the hypertonic solution may contain one or more enzymes, preferably aprotinin, or enzymes from the group of nucleases, e.g. aprotinin or DNase type I/II and/or RNase in an amount of from 1 KIU or U/ml to 100 KIU or U/ml preferably 10 KIU or U/ml.

Hypertonic solutions can be used before or after the hypotonic solution - the essence of their use is to achieve effective decellularization. Rinsing with a washing solution, preferably PBS or distilled water or a mixture thereof in a ratio of 1:1 to 1:4, respectively, may be used at each change of the decellularizing solution.

The purpose of the subsequent steps is to wash out residual decellularizing agents from the extracellular matrix. For this purpose, a washing buffer is instilled into the bladder with a neutral or mild alkaline pH in the range of 7 to 9, preferably 7.6, in the form of Tris buffer with the addition of magnesium and/or sodium and/or calcium chlorides at a concentration of 0.1 mM to 500mM preferably 150mM for 2 hours to 48 hours at a flow rate of 1 liter/hour to 700 liters/hour preferably 300 liters/hour.

The decellularized bladder (acellular BAM) is then rinsed in distilled water or PBS 1x for a period of 1 to 20 days, preferably 14 days.

Then, in the case of decellularization of the entire bladder, the body of the bladder is removed by cutting off the part with the ureters and urethra, and the extracellular matrix (BAM) obtained in this way is subjected to the lyophilization process. For this purpose, the BAM matrix is pre-frozen at a temperature of -5 to -150°C, preferably -20°C for 1 to 12 hours, preferably 2 hours, then dried at a pressure of 6.110 mbar to 0.0010 mbar, most preferably 0.120 mbar, at a temperature of 0°C from -100°C, most preferably -60°C, for 2 to 24 hours, most preferably 17 hours, and again dried at a pressure of 1 to 2000 mbar, most preferably 1,000 mbar, for 1 to 12 hours, most preferably for 2 hours, at 0°C from -100°C, most preferably -60°C.

The produced freeze-dried BAM matrix is sealed with a polymer and cross-linked. For the purpose of cross-linking, a natural dialdehyde polymer is applied to its surface using a static method consisting in immersing the composite or a dynamic method - spraying, coating or surfacing and coating, a natural dialdehyde polymer preferably at a mass concentration of 0.1 to 5%, most preferably 1%, in PBS from 10x to 1x, preferably 1x, heated to change the conformation of the collagen polymer chains, which facilitates the penetration of the polymer into the BAM, preferably in a vacuum dryer at a pressure of 0.5 [kg/m²] to 1 [kg/m²], preferably 0.8 [kg/m²] m²], at a temperature of 0 to 40°C, preferably 37°C, for 2 to 72 hours, preferably 48 hours.

Then, in order to increase the durability of the bonds between the BAM matrix and the polymers - collagen and cellulose, a sodium borohydride solution of 0.1 to 2%, preferably 1%, dissolved in ethanol at a concentration of 90 to 99%, preferably 96%. The reaction takes place for 1 to 5 hours, preferably for 3 hours at a temperature of 0 to 10°C, preferably 4°C. In order to bind non-crosslinked functional groups, a 0.1M glycine solution is added to its surface by applying it.

Then, in order to increase the durability of the bonds between the BAM matrix and polymers - collagen and cellulose, a solution of sodium borohydride from 0.1 to 2%, preferably 1%, dissolved in ethanol with a concentration of 90 to 99%, preferably 96%, can be added to the obtained BAM composite (by pouring it onto the BAM surface) to consolidate the cross-linking. The reaction takes place for 1 to 5 hours, preferably for 3 hours at a temperature of 0°C to 10°C, preferably 4°C. In order to bind non-crosslinked functional groups, a 0.1M glycine solution is added by applying it to its surface.

After the cross-linking process is completed, the ready implant is subjected to a washing process in a PBS solution, preferably from 1x to 10x, most preferably 1x, from 2 to 10 times, preferably 5 times. For this purpose, the implant is placed in a polystyrene petri dish and PBS is added until the implant is completely covered and incubated.

Then the implant is frozen preferably at a temperature of -5°C to -150°C, most preferably -20°C for 1 to 12 hours, preferably 2 hours, preferably dried at a pressure of 6.110 mbar to 0.0010 mbar at a temperature of 0°C to -100°C during 2 to 24h, preferably 0.120 mbar at -60°C for 17 hours, and finally dried preferably at a pressure of 1 to 2000 mbar for 1 to 12 hours, most preferably 1000 mbar for 2 hours.

The implant is sterilized with ethylene oxide, preferably at a concentration of 1500mg/m3, during exposure from 5 to 20 hours, preferably 6 hours, at a vacuum of max. -0.095MPa.

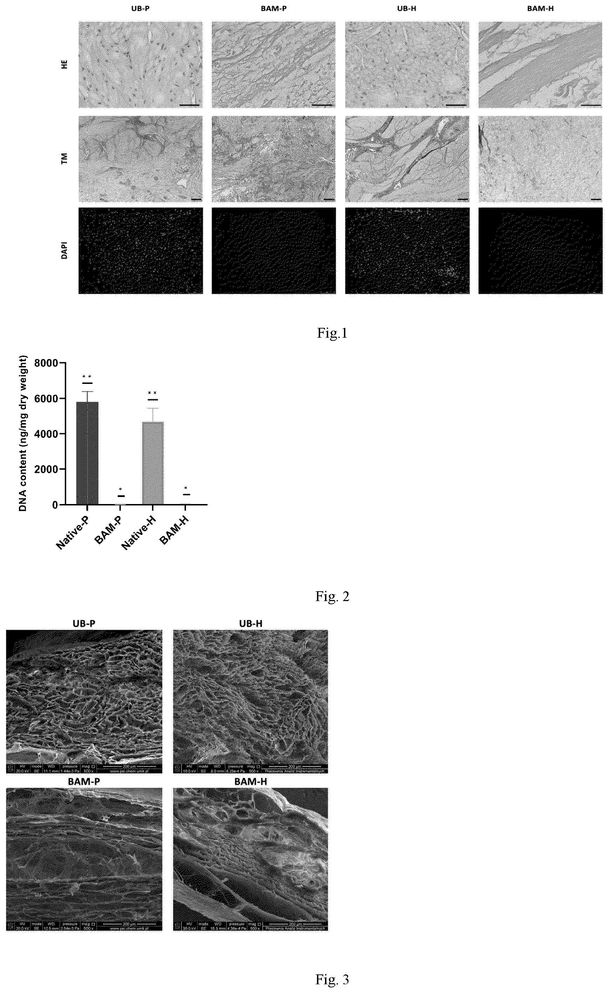

The BAM matrix from which the implant is made is completely devoid of cells and their remains. Histological and immunofluorescent staining of the BAM matrix showed no visible cell nuclei, as shown in Fig.1. The DNA content of the BAM matrix is less than 50 ng/mg of dry tissue - 20.49±16.57ng/mg, as shown in Fig.2.

Fig.1 Analysis of the acellularity of the BAM scaffold; HE stain, Masson's Trichrome, DAPI. UB-P -Urinary Bladder-Porcine; BAM-P - Bladder Acellular Matrix-Porcine; UB-H - Urinary Bladder-Human; BAM-H - Bladder Acellular Matrix-Human.

Fig.2 Comparison of the concentration of DNA isolated from the porcine bladder wall (Native-P), porcine BAM matrix (BAM-P) and human bladder (Native-H) and human BAM matrix (BAM-H).

Scanning electron microscopy (SEM) analysis confirmed the acellularity of the BAM scaffolds. It was shown that the obtained BAM scaffolds were characterized by high porosity. The structure of collagen fibers was preserved. The outer layer of collagen fibers was not affected - the fibers were smooth, which proves that the decellularization process does not damage the structural fibers of the extracellular matrix (Fig. 3).

Fig.3 SEM analysis of bladder structure and BAM scaffold; UB-P - Urinary Baldder-Porcine; BAM-P - Bladder Acellular Matrix-Porcine; UB-H - Urinary Bladder-Human; BAM-H - Bladder Acellular Matrix-Human.

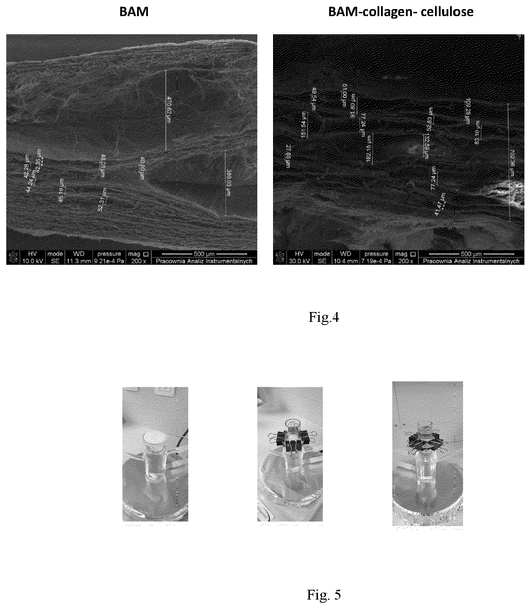

The implant according to the present invention is impermeable to urine and retains properties similar to the native bladder wall. The main purpose of sealing BAM with polymers, e.g. collagen and cellulose, is to create an insulating layer for urine, which adversely affects the regenerative processes in the reconstructed bladder until the urothelial epithelium is restored. Scanning electron microscopy (SEM) analysis showed that the polymer-sealed BAM matrix had a smaller pore size (60,723±58,280µm) than BAM (207±177µm) (Fig.4).

Fig.4 SEM analysis of the structure of the BAM matrix - cell-free bladder matrix and the implant - BAM matrix sealed with collagen and cellulose polymers.

In addition, the graft permeability was assessed using the Franz chamber (Fig.5). In order to best imitate the conditions in the bladder, the measurement was carried out at a temperature of 38°C. The level of urine permeation through the BAM-collagen-cellulose composite was assessed by changes in fluorescence (fex=310nm; fem=430nm), which were measured using a Varioskan^{™} LUX (Thermo Scientific, USA). The study showed that the implant (BAM composite sealed with polymers) retains tightness for at least 24 hours.

Fig.5 Evaluation of implant permeability (BAM-collagen-cellulose composite) using a Franz chamber.

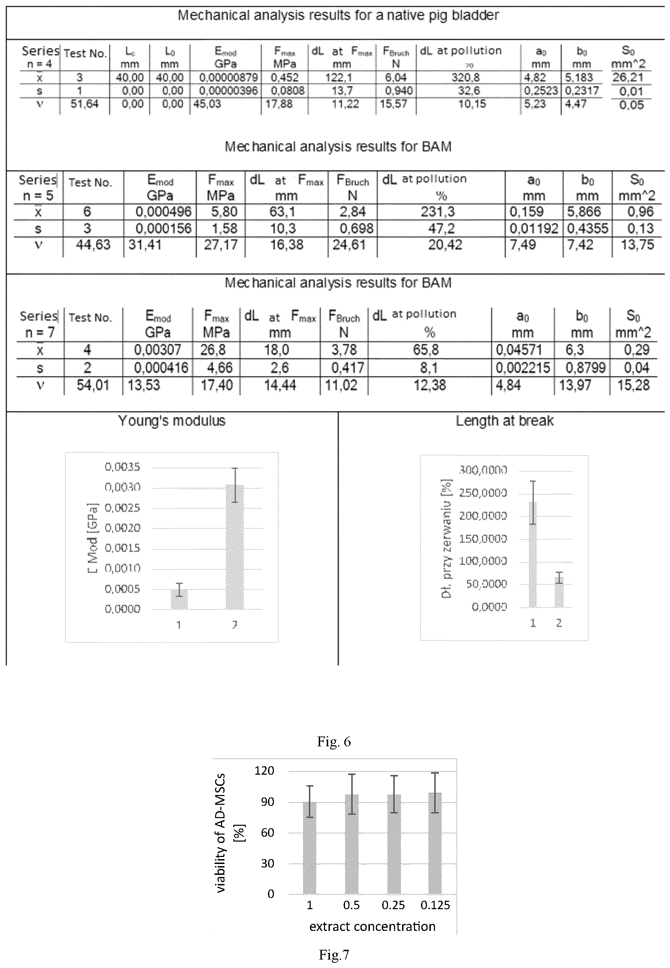

Mechanical analysis proved that the obtained composites are flexible, which is proved by Young's modulus (0.00307±0.000416) and elongation at break (65.8±8.1). The Young's modulus for the native pig bladder was 0.00000879 ±0.00000396, which means that the obtained composite is less flexible than the native pig bladder (Fig.6), but comparing both products to another polymer, which is polyethylene, for which the modulus Young's is from 0.1 to 0.4 GPa, both BAM and BAM sealed - implant, according to the invention - are characterized by a high degree of flexibility.

Fig.6 Results of the mechanical analysis of the native bladder wall, BAM - acellular matrix from the bladder and the implant - acellular matrix from the bladder sealed with polymers. 1-BAM; 2 - implant (BAM sealed with collagen and cellulose)

Conducted in vitro and in vivo studies have showed that the implant of the present invention is biocompatible - non-cytotoxic, non-genotoxic, non-inducing systemic toxicity and intradermal irritation/reactivity.

Cytotoxicity assessment according to ISO 10993-5:2009 of lyophilized cell-free bladder matrix.

Cytotoxicity studies of the prepared cell-free porcine bladder matrices were performed using the MTT test. MTT analyzes were performed on mesenchymal stromal cells derived from porcine adipose tissue (AD-MSCs). Cell viability after 24-hour exposure to extracts prepared from lyophilized BAMs was 90.5% ± 15.6% for 100% extract, which means no cytotoxic effect (Fig.7).

Fig.7. Viability of porcine AD-MSCs incubated with different concentrations of BAM extract made from porcine bladder.

### Cytotoxicity analysis of the implant according to the invention (freeze-dried cell-free bladder matrix - polymer composite)

The cytotoxicity assessment service of the implant according to the invention was performed in accordance with the ISO 10993-5 standard in an accredited external laboratory. In accordance with ISO 10993-12:2021(E), an extract from the implant was prepared in culture medium by immersing the implant in the medium and extracted for 24 hours at 37°C. After extraction, the positive control latex (K+), negative HDPE (K-) and test implant extracts were clear, with no noticeable particles.

L929 cells were incubated with the graft extracts at 37°C and 5% CO₂ for 24 hours. Cytotoxicity was assessed using a trypan blue test.

Achieving a numerical grade of 3 or greater is considered cytotoxic.

Acceptance criteria: blank < 3; positive control grade ≥ 3; negative control grade < 3; extract from the tested item grade < 3 (Tab.1).

**Tab.1. Cytotoxic potential assessment results.**

| Sample | Grade | |
|---|---|---|
| Blank | 0 | Valid |
| Negative control | 0 | Valid |
| Positive control | 4 | Valid |
| Implant according to the invention | 0 | Non-cytotoxic |

Based on the results interpreted according to ISO 10993-5:2009(E), the implant according to the invention should be considered non-cytotoxic.

Analysis of the genotoxicity of the implant according to the invention in terms of mutagenic activity

The evaluation of the genotoxicity of the implant according to the invention was carried out using the AMESA test in accordance with the ISO 10993-5 standard in an accredited external laboratory.

**Tab.2. AMESA test results.**

| **Strain** | **Without S9** | | | | **With S9** | | | |
|---|---|---|---|---|---|---|---|---|
| | Baseline | Fold increase over baseline | Binominal B-value | Results | Baseline | Fold increase over baseline | Binominal B-value | Results |
| TA98 | 9,29 | 1,44 | 1,0000 | PASS | 10,65 | 0,19 | 0,0000 | PASS |
| TA100 | 7,86 | 0,25 | 0,0003 | PASS | 7,55 | 0,53 | 0,4579 | PASS |
| TA1535 | 2,49 | 0,27 | 0,2340 | PASS | 1,00 | 1,67 | 0,9994 | PASS |
| TA1537 | 1,00 | 0,33 | 0,7358 | PASS | 1,24 | 1,34 | 0,9842 | PASS |
| *E.Coli* WP2 uvrA [pKM101] | 12,18 | 1,15 | 0,9964 | PASS | 12,73 | 1,39 | 0,9999 | PASS |

**Tab.3. Summary of AMESA test results.**

| **Strain** | **Mutagenic data points** | | **Overall Result for sample** | **Solvent control** | | **Positive control** | |
|---|---|---|---|---|---|---|---|
| | **w/o S9** | **with S9** | | **w/o S9** | **with S9** | **w/o S9** | **with S9** |
| **TA98** | No | No | Probably not mutagenic | PASS | PASS | PASS | PASS |
| **TA100** | No | No | Probably not mutagenic | PASS | PASS | PASS | PASS |
| **TA1535** | No | No | Probably not mutagenic | PASS | PASS | PASS | PASS |
| **TA1537** | No | No | Probably not mutagenic | PASS | PASS | PASS | PASS |
| **E. coli uvrA [pKM101]** | No | No | Probably not mutagenic | PASS | PASS | PASS | PASS |

The binomial value B indicates the probability of a spontaneous mutational event. A binomial value of B ≥ 0.99 indicates that the odds are ≤ 1% that this result is due to a spontaneous mutation. If both a fold growth ≥ 2 or 3 (strain dependent) and a binomial B value ≥ 0.99 are present for the test sample under the specified conditions (strain, +/- fraction S9), this is noted as "yes" under the "mutagenic entry data points" (Tab.2, Tab.3).

Based on the results interpreted in accordance with ISO 10993-3:2014(E), the implant according to the invention should be considered non-mutagenic in the tested species.

### Systemic toxicity study of the implant according to the invention

The study was carried out on a mouse model by administering extracts from the implant according to the invention, in accordance with the PN-EN ISO 10993-11 standard in an accredited external laboratory. The test used 4 groups of 5 animals. Groups of animals were injected with an extract of 50 ml/kg with a polar solvent (sodium chloride), a non-polar solvent (cotton oil), a polar solvent and a non-polar control. Non-polar extract (cotton oil), polar extracts (sodium chloride), control of polar and non-polar solvents were injected intraperitoneally. Animals were clinically examined and weighed 24±2 hours, 48±2 hours, 72±2 hours after injection. Animals were euthanized 72±2 hours after injection.

None of the test or control animals and animals in the supplementary group showed overt signs of toxicity. Common clinical signs and observations, ISO 10993-.11:2017(E), at each observation point. During the observation period, none of the animals treated with the extract of the implant of the invention showed significantly greater biological reactivity than the animals treated with the control, none of the animals died, none of the animals lost 10% or more of their body weight.

According to ISO 10993-11:2017(E), the extracts of the implant of the invention showed no potential for acute systemic toxicity with intraperitoneal and intravenous administration.

### Evaluation of the intradermal irritation/reactivity of the implant according to the invention

One group of 3 rabbits was used in the study. The left side of each rabbit was treated with extracts from the implant of the invention and the right side was treated with a relative solvent control (sodium chloride and cottonseed oil).

All animals were observed immediately after injection and 24±2, 48±2 and 72±2 hours after injection to assess for signs of local reaction. Injection sites were examined for any tissue reactions such as erythema, edema.

**Tab.4. Intradermal irritation/reactivity results for extracts in a polar solvent**

| EXTRACT IN SODIUM CHLORIDE INJECTION | | | | | | | | | |
|---|---|---|---|---|---|---|---|---|---|
| Rabbit No. | Site No. | 24±2 hour after injection | | 48±2 hour after injection | | 72±2 hour after injection | | PIS | PIS total |
| | | E | O | E | O | E | O | | |
| 1/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |
| 2/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | 0 |
| | 4 | 0 | 0 | 0 | 0 | 0 | 0 | | |
| | 5 | 0 | 0 | 0 | 0 | 0 | 0 | | |
| 3/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |

| SOLVENT CONTROL: SODIUM CHLORIDE INJECTION | | | | | | | | | |
|---|---|---|---|---|---|---|---|---|---|
| Rabbit No. | Site No. | 24±2 hour after injection | | 48±2 hour after injection | | 72±2 hour after injection | | PIS | PIS total |
| | | E | O | E | O | E | O | | |
| 1/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |
| 2/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | 0 |
| | 4 | 0 | 0 | 0 | 0 | 0 | 0 | | |
| | 5 | 0 | 0 | 0 | 0 | 0 | 0 | | |
| 3/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |

| | | | | | | | | | |
|---|---|---|---|---|---|---|---|---|---|
| E = Erythema O = Oedema PIS = Primary Irritation Score | | | | | | | | | |

**Tab.5. Intradermal irritation/reactivity results for extracts in a non-polar solvent**

| EXTRACT IN COTTONSEED OIL | | | | | | | | | |
|---|---|---|---|---|---|---|---|---|---|
| Rabbit No. | Site No. | 24±2 hour after injection | | 48±2 hour after injection | | 72±2 hour after injection | | PIS | PIS total |
| | | E | O | E | O | E | O | | |
| 1/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |
| *2*/*21* | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | 0 |
| | 4 | 0 | 0 | 0 | 0 | 0 | 0 | | |
| | 5 | 0 | 0 | 0 | 0 | 0 | 0 | | |
| 3/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |

| SOLVENT CONTROL: COTTONSEED OIL | | | | | | | | | |
|---|---|---|---|---|---|---|---|---|---|
| Rabbit No. | Site No. | 24±2 hour after injection | | 48±2 hour after injection | | 72±2 hour after injection | | PIS | PIS total |
| | | E | O | E | O | E | O | | |
| 1/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |
| 2/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | 0 |
| | 4 | 0 | 0 | 0 | 0 | 0 | 0 | | |
| | 5 | 0 | 0 | 0 | 0 | 0 | 0 | | |
| 3/21 | 1 | 0 | 0 | 0 | 0 | 0 | 0 | 0 | |
| | 2 | 0 | 0 | 0 | 0 | 0 | 0 | | |
| | 3 | 0 | 0 | 0 | 0 | 0 | 0 | | |
| | 4 | 0 | 0 | 0 | 0 | 0 | 0 | | |
| | 5 | 0 | 0 | 0 | 0 | 0 | 0 | | |

| | | | | | | | | | |
|---|---|---|---|---|---|---|---|---|---|
| E = Erythema O = Oedema PIS = Primary Imitation Score | | | | | | | | | |

During the study, all sites treated with the extract of the implant according to the invention showed no signs of erythema, discoloration or edema 24±2 hours, 48±2 hours and 72±2 hours after injection (Tab.4, Tab.5).

Based on the results, interpreted according to ISO 10993-10:2021(E), the implant of the invention does not cause skin irritation.

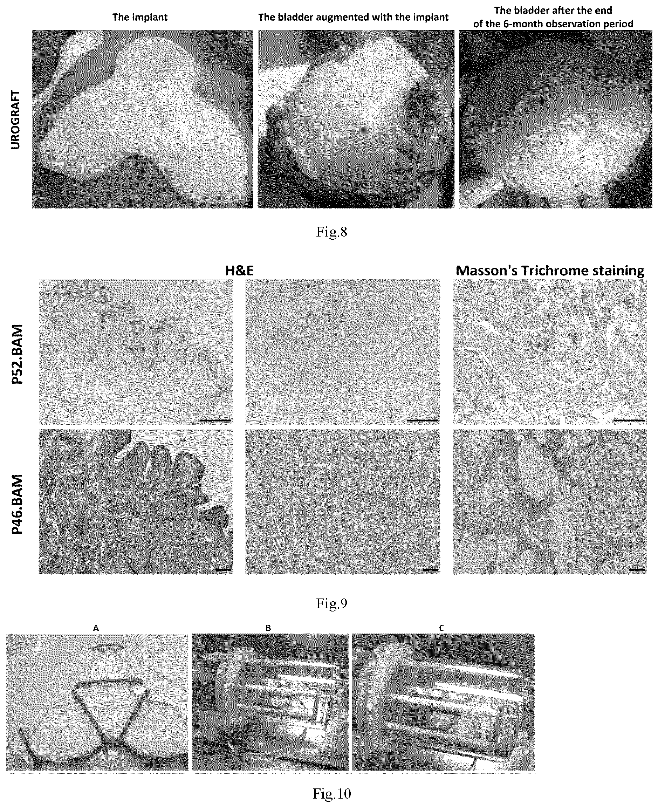

In order to assess the possibility of reconstructing the bladder wall using the implant according to the invention, 20 pigs were operated on. A three-armed configuration with an area of 30 cm² was implanted, a circular-shaped implant with the same area was used as a control. The observation period lasted 6 months (Tab.6). Macroscopic and photographic analysis of the implant on the filled bladder was performed. The implanted graft was very well integrated with the recipient's native tissue, macroscopically the implant was identical to the native bladder, the border of the implant was not visible. No adhesions with the intestine were observed. A small Y-shaped scar corresponding to the configuration of the implanted biomaterial was observed macroscopically. A well-developed mucosa was observed on the inner surface of the reconstructed area (Fig.8). Histological analysis using HE staining showed regeneration of the multilayered urothelial epithelium, smooth muscles and blood vessels. Trichrom-Masson staining confirmed the regeneration of the muscular layer. In the central part, there were places where the regeneration process was less advanced compared to the arms, the muscle fibers were smaller and less organized (Fig.9). The regeneration process of the reconstructed bladder wall using the implant according to the invention was more intense and was characterized by a much smaller scarring area compared to the control group in which the circular implant was used.

Fig.8. A porcine bladder reconstructed by tissue engineering techniques using an implant according to the invention. The photos show: the implant, the bladder augmented with the implant, and the bladder after the end of the 6-month observation period.

Fig.9 Histological analysis of the tissue-engineered bladder wall after 6 months of observation. The photos were taken from a fragment of the arm of the implant according to the invention. Visible regeneration of the urothelial epithelium and smooth muscle layer. Staining with H&E and Masson's Trichrome. Lens magnification 10x.

**Tab.6. Data on preclinical studies conducted so far on a pig model. One recorded animal death was unrelated to the study implant. AD-MSCs - adipose derived mesenchymal stromal cells.**

| | |
|---|---|
| Number of pigs operated on | 20 |
| Implant according to the invention | 7 |
| Implant according to the invention seeded with AD-MSCs | 7 |
| Circle-shaped implant | 3 |
| Implant-shaped BAM matrix according to the invention without modification | 3 |
| Observation period ended | 12 |
| Deaths before the end of the follow-up period | 1 |
| Survivability | 95% |

### Cell seeding on an implant according to the invention

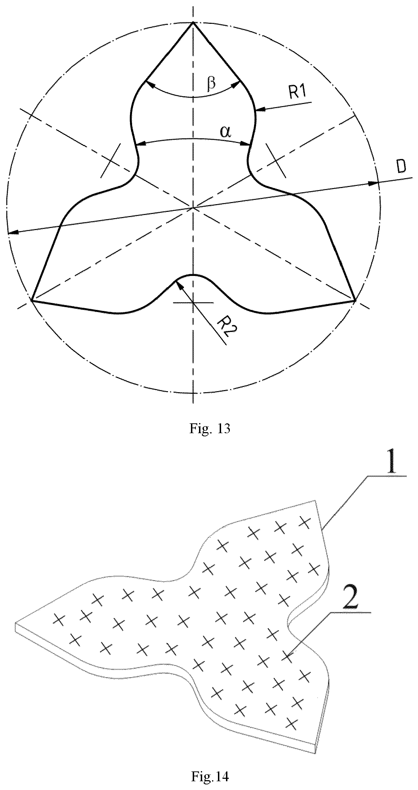

Human AD-MSCs were seeded onto the implant according to the invention by injecting the cells into the material from the outside at a density of 10 million/cm². The cells on the matrix were cultured for 7 days in a bioreactor (Fig.13). During the cultivation, the culture medium was changed daily. After completion of the culture, the implant according to the invention seeded with cells was fixed in a solution of 2.5% glutaraldehyde + 2% formaldehyde and subjected to SEM analysis. It has been shown that AD-MSCs cells seeded on the implant according to the invention remain viable, attach and proliferate not only on its surface and deep into the 3D structure of the implant (Fig.9).

Fig.10. Cultivation in a bioreactor. A - implant according to the invention, after seeding with AD-MSCs cells, placed in a dedicated rack, B - rack with implant placed in a bioreactor, C - implant after 7 days of culture in a bioreactor.

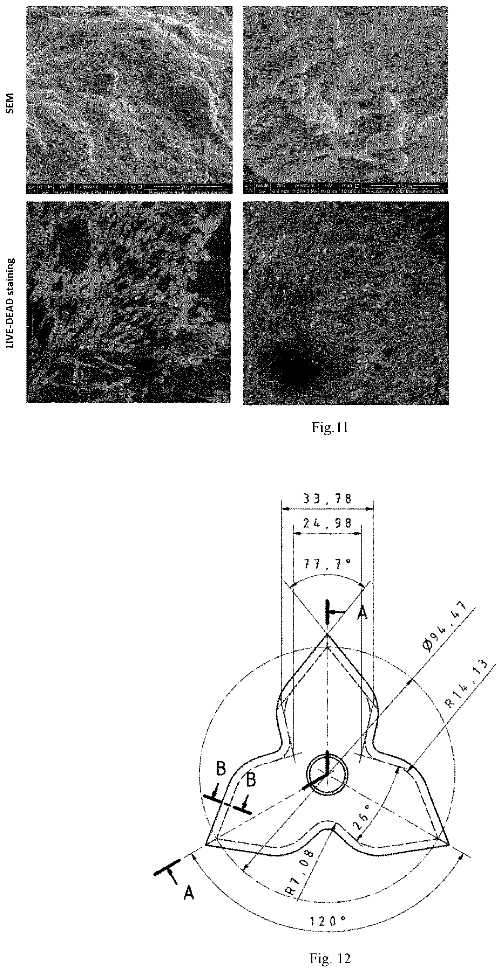

Fig.11. Human AD-MSCs seeded on the implant according to the invention - pictures from SEM and from live-dead staining. Image from a confocal microscope.

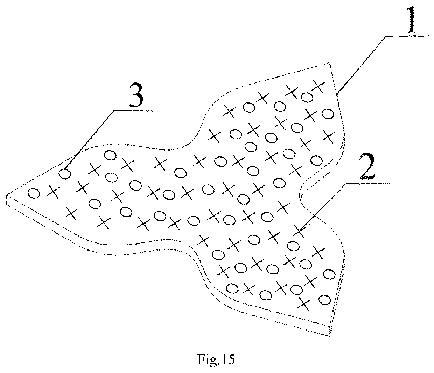

The invention is presented in the implementation examples and in the drawing, where Fig. 12 shows the shape of the implant, Fig. 13 shows a schematic view of the implant structure, and Fig. 14 and 15 show the implant according to the invention seeded with cells.

### Example I

The bladder is subjected to dynamic decellularization, during which the decellularizing solution is pumped into the bladder. The bladder is flushed with a washing buffer, PBS with EDTA, to flush out residual residual urine. For initial cell lysis, the bladder is frozen and thawed three times at -80°C and 37°C. The first solution used during decellularization is an enzyme solution containing 0.25% trypsin, which is used for 2 hours. This is followed by decellularization using a mildly alkaline hypotonic Tris buffer solution containing active amounts of the proteolytic inhibitor EDTA and hypertonic solutions which contain a detergent and containing active amounts of the proteolytic inhibitors, Tris buffer with 1% Triton X-100 and Tris buffer with 1% SDS - for 72 and 24 hours, respectively. One enzyme aprotinin at a concentration of 10 KIU/ml is added to the decellularizing solutions. In other embodiments, hypertonic solutions may be used before or after the hypotonic solution. The last step is to use a mildly alkaline washing buffer in the form of Tris buffer with the addition of 2mM magnesium chloride and calcium chloride and 150mM sodium chloride. Washing with PBS is used with each change of the decellularizing solution. Following decellularization and rinsing of the decellularized tissue in PBS 1x for 14 days, a cell-free Bladder Acellular Matrix (BAM) is formed. Then, the body of the bladder is removed by cutting off the part with the ureters and urethra, and the extracellular matrix is subjected to a lyophilization process - initial freezing of the matrix at -20°C for 2 hours; then drying at 0.120 mbar at -60°C for 17 hours and final drying at 1000 mbar for 2 hours.

The shape of the implant is cut out from the lyophilized matrix using a punch.

The shape of the implant is an axially symmetrical, three-armed implant with arms resembling a lily petal inscribed in a circle with an area of 30 cm², described inside with a diameter D of 9.45 cm and a thickness of 0.5 cm, with an angle of opening alpha of 26°, radius R1 of 14.3 mm and a radius of R2 7.08 mm, and a beta point angle of 77.7°.

The implant is immersed with the outer side down in a previously prepared solution of type I collagen with a mass concentration of 1%, in 0.17M hydrochloric acid in a dedicated form and transferred to a vacuum dryer at a pressure of 0.8 [kg/m²] at a temperature of 37°C for 24 hours. Then, in order to cross-link in static conditions, a solution of dialdehyde cellulose with a mass concentration of 1% in PBS 1x is applied to the obtained composite and incubated in a vacuum dryer at a pressure of 0.8 [kg/m²] at a temperature of 37°C for 48 hours . Then, in order to increase the durability of the bonds between the BAM and polymers / in order to consolidate the cross-linking, a 1% solution of sodium borohydride dissolved in 96% ethanol is added to the obtained composite. The reaction proceeds for 3 hours at 4°C. In order to bind non-crosslinked functional groups, a 0.1 M glycine solution is added to the composite for 2 hours. After the cross-linking process is completed, the implant is subjected to the process of rinsing in a 1x PBS solution at least 5 times, and then freezing at -20°C for 2 hours and freeze-drying in the vacuum range of 0.1 mbar for 17 hours and final drying at a pressure of 1000 mbar for 2 hours. The implant is sterilized with ethylene oxide, at a concentration of 1500mg/m³ for 6 hours, at a vacuum of max. -0.095MPa.

As a result of the example, an implant was obtained consisting of a cell-free matrix made of the bladder wall (BAM), collagen and cellulose.

### Example II.

A fragment of the bladder is subjected to dynamic decellularization. The bladder is flushed with a washing buffer, PBS with EDTA, to flush out residual residual urine. The first solution used during decellularization is an enzyme solution of 0.5% trypsin for 1 hour. This is followed by decellularization using a hypotonic solution - Tris buffer with a mild alkaline pH, which contains active amounts of proteolytic agent inhibitor - 5mM EDTA. A hypertonic-Tris buffer solution is then used that contains Tris buffer detergent with 1% SDS for 72 hours.

DNase type I is added to the decellularizing solutions. The last step is to use a mildly alkaline wash buffer, Tris buffer with 2mM magnesium chloride and calcium chloride and 150mM sodium chloride.

Each time the decellularizing solution is changed, a washing solution, in the form of PBS, is rinsed. As a result of the performed decellularization and the process of rinsing the decellularized tissue in PBS 1x alternating with distilled water for a period of 21 days. A cell-free BAM made from the bladder wall (Bladder Acellular Matrix, BAM) is formed. The extracellular matrix is subjected to a lyophilization process - initial freezing of the matrix at -20oC for 2 hours; drying at 0.120 mbar at -60°C for 17 hours and final drying at 1000 mbar for 2 hours. The shape of the implant is cut out from the freeze-dried matrix using a punch.

The shape of the implant is an axially symmetrical, three-armed implant with arms resembling a lily petal inscribed in a circle with an area of 50 cm2, described inside with a diameter D of 12.2 cm and a thickness of 0.4 cm, with an angle of opening alpha of 33°, radius R1 18.46 mm and radius R2 9.14 mm, and a 100° beta point angle.

The implant is covered by spraying with a previously prepared collagen solution with a mass concentration of 1% in 0.17M hydrochloric acid and incubated at 37°C for 36 hours. The BAM-collagen composite is then spray coated with 1% dialdehyde cellulose in 1× PBS and incubated at 37°C for 50 hours. Then, in order to increase the durability of the bonds between the BAM and polymers / to consolidate the cross-linking, the obtained composite is immersed in a sodium borohydride solution of 1% sodium borohydride dissolved in 96% ethanol. The reaction proceeded for 3 hours at 4°C. After the cross-linking process is completed, the implant is subjected to the process of rinsing in a 1x PBS solution 5 times, and then freezing at -20°C for 2 hours and freeze drying in the vacuum range from 0.1 mbar to 0.120 mbar for 17 hours and final drying under a pressure of 1000 mbar for 2 hours. The implant is sterilized with ethylene oxide, at a concentration of 1500mg/m³ for 6 hours, at a vacuum of max. -0.095MPa.

As a result of the example, an implant was obtained consisting of an acellular matrix made of the bladder wall (BAM), collagen and cellulose.

### Example III

An implant was prepared as in Example 1, except that instead of being sealed with collagen and cross-linked with cellulose, the lyophilized cell-free matrix was sealed with 1% collagen cross-linked with a 5 mM solution of genipin dissolved in distilled water.

As a result of the example, an implant was obtained consisting of a cell-free matrix made of the bladder wall (BAM) and collagen.

### Example IV

The implant was obtained as in example 1. Additionally, on the sterilized external part of the implant in clean conditions in cleanroom laboratories, previously cultured mesenchymal stromal/stem cells isolated from adipose tissue collected during the liposuction procedure are seeded at a density of 10 x 10⁶ cells/cm² by puncturing of the implant to a depth of 2 mm and injecting the cell suspension. The implant is then placed in a bioreactor and incubated for 7 days in an atmosphere of 5% CO₂ and a temperature of 37°C. The implant prepared in this way can be implanted immediately after preparation or stored at -150°C until use. For this purpose, the implant should be frozen in a cryoprotective medium with the addition of 10% DMSO.

The example resulted in an implant consisting of a cell-free bladder wall matrix (BAM), collagen, cellulose and adipose tissue mesenchymal stromal/stem cells.

### Example V

The implant was obtained as in example 1. In cleanroom laboratories, the sterilized implant is impregnated with cross-linked heparin and growth factors, VEGF. For this purpose, the implant is incubated in a solution of heparin sodium with the addition of M1-ethyl-3-(3-dimethylaminopropyl) carbodiimide (EDC) and N-hydroxysuccinimide (NHS) for 16 hours and then incubated for 12 hours at 4°C at 0.09% sodium chloride solution with VEGF at a concentration of 2µg/ml.

As a result of the example, an implant was obtained consisting of a cell-free matrix made of bladder wall (BAM), collagen, cellulose and VEGF.

### Example VI

An implant was obtained as in example 5. Additionally, in cleanroom laboratories, the sterilized implant is impregnated with preferably cross-linked heparin and growth factors, VEGF, by immersing the matrix in a solution of growth factor medium. Then, from the outer part, in clean conditions in cleanroom laboratories, previously cultured mesenchymal stromal/stem cells isolated from adipose tissue collected during liposuction, with a density of 10 x 10⁶ cells/cm², are seeded by puncturing the implant to a depth of 2 mm and injecting a cell suspension . The implant is then placed in a bioreactor and incubated for 7 days in an atmosphere of 5% CO₂ and a temperature of 37°C. The implant prepared in this way can be implanted immediately after preparation or stored at -150°C until use. For this purpose, the implant should be frozen in a cryoprotective medium with the addition of 10% DMSO.

The example resulted in an implant consisting of a cell-free bladder wall matrix (BAM), collagen, cellulose, VEGF and adipose tissue mesenchymal stromal/stem cells.

### Example VII.

The bladder reconstruction implant has the form of a five-arm, axisymmetric element with arms inscribed in a circle and is made of an acellular matrix cross-linked with a polymer, the acellular matrix being made of a decellularized human bladder. The polymer is type I collagen.

### Example VIII.

Example VIII differs from Example VI in that the implant is in the form of an axially symmetrical four-armed element, the polymer being type II collagen.

In other embodiments, the polymer may also be collagen type III or IV of animal origin or cellulose or collagen with cellulose in a ratio of 1:1 or gelatin or starch or natural silk or cellulose esters and ethers in the form of carboxymethyl cellulose or carboxyethyl cellulose or methyl cellulose or chitosan esters and ethers in the form of N or O-carboxymethyl chitosan or O-methyl carboxy chitosan, or hyaluronic acid or fibrinogen or a synthetic polymer soluble in organic solvents in the form of poly(lactide) or poly(glycolide) or poly(caprolactone) or polyamide or polyimide or poly(trimethylene carbonate) or poly(dioxanone) or poly(vinyl acetate) or poly(vinyl alcohol) or poly(N-vinyl-2-pyrrolidone) or polyurethane or poly(ether urethane) or polycarbonate or aromatic polycarbonate or poly(ethylene oxide) or poly(glycol ethylene) or poly(acrylic acid) or poly(acrylamide) or poly(acrylonitrile) or poly(chloride) u vinyl) or a 1:1 mixture thereof or a copolymer in the form of L-lactide or caprolactone or poly(L-lactide-co-caprolactone).

### Example IX

The implant is an axisymmetric, three-armed implant with arms resembling a lily petal inscribed in a circle with an area of 3 cm², described inside with a diameter D of 2.99 cm and a thickness of 0.2 cm, with an angle of 8° alpha, radius R1 4.52 mm and radius R2 2.23 mm, and a beta point angle of 24.6°. The cell-free matrix is made from decellularized porcine bladder. The polymer is collagen with cellulose in a 1:1 ratio. The matrix contains inside mesenchymal stromal cells isolated from the bone marrow with a density of 10 x 10⁶ cells/cm².

In other embodiments, the mesenchymal stromal cells are isolated from subcutaneous adipose tissue or Wharton's jelly or other vascularized human tissues. The cell density will be from 1x10⁶ to 100x10⁶ at each point.

### Example X

The implant is an axisymmetric, three-armed implant with arms resembling a lily petal inscribed in a circle with an area of 60 cm², described inside a diameter of D 14 cm and a thickness of 1 cm, with an angle of 37° alpha, radius R1 20.5 mm and radius R2 10 mm, and apex angle beta of 110°. The cell-free matrix is made from decellularized porcine bladder. The polymer is type I collagen. Inside, the matrix contains mesenchymal stromal cells isolated from the subcutaneous adipose tissue with a density of 1 x 10⁶ cells/cm².

### Example XI.

The scaffold for the bladder reconstruction implant is a polymer cross-linked acellular matrix, wherein the acellular matrix is made of a decellularized fragment of the bladder and the polymer is type I collagen.

### Example XII.

The scaffold for the bladder reconstruction implant is an acellular matrix cross-linked with a polymer, wherein the acellular matrix is made of a decellularized portion of the intestinal wall and the polymer is type II collagen. The cell-free matrix contains intracellular mesenchymal stromal cells isolated from subcutaneous adipose tissue at a density of 10x10⁶ cells/cm².

In other embodiments, the polymers may be type III or IV collagen or cellulose or collagen with cellulose in a ratio of 0.1:2, preferably 1:1 or gelatin or starch or natural silk or cellulose esters and ethers preferably carboxymethyl cellulose or carboxyethyl cellulose or methyl cellulose, or chitosan esters and ethers preferably N or O-carboxymethyl chitosan or O-methyl carboxy chitosan or hyaluronic acid or fibrinogen or a synthetic polymer soluble in organic solvents preferably poly(lactide) or poly(glycolide) or poly(caprolactone) or polyamide or polyimide or poly(carbonate) trimethylene) or poly(dioxanone) or poly(vinyl acetate) or poly(vinyl alcohol) or poly(N-vinyl-2-pyrrolidone) or polyurethane or poly(etherurethane) or polycarbonate or aromatic polycarbonate or poly(ethylene oxide) or polyethylene (ethylene glycol) or poly(acrylic acid) or poly(acrylamide) or poly(acrylonitrile) or poly(vinyl chloride) or a mixture thereof in a ratio of 0.1:2 to 2:1 or k an opolymer of preferably L-lactide and caprolactone or poly(L-lactide-co-caprolactone).

In terms of cross-linking, in other embodiments, mesenchymal stromal cells can also be isolated from bone marrow or Wharton's jelly or other vascularized human tissues. The cell density will be from 1x10⁶ to 100x10⁶ at each point.

### Example XIII.

The method of obtaining a bladder reconstruction implant is that the human bladder is rinsed with a washing solution and subjected to decellularization, and the thus obtained acellular matrix is sealed by dipping, spraying, padding or coating, in an embodiment by dipping, with a polymer in the form of type I collagen, with a polymer mass concentration of 0.5 to 2%, in an embodiment 1% in hydrochloric acid with a molar concentration of 0.01M to 0.05M, in an embodiment 0.17M, and dried, and then the composite thus obtained BAM is crosslinked by dipping, spraying, padding or coating with a crosslinking solution, in an embodiment with a dialdehyde natural polymer in the form of resorbable dialdehyde cellulose with a mass concentration of 0.1 to 5%, in an embodiment 1% and in PBS from 10x to 1x, in embodiment 1x, heats, places in a petri dish and adds PBS solution until completely covered and the BAM composite and incubated, then re-frozen, dried and sterilized with ethylene oxide, and the matrix thus obtained is punched to the desired shape.

### Example XIV

The method of obtaining an implant for bladder reconstruction is that the bladder obtained from a pig is rinsed with a washing solution in the form of PBS and subjected to decellularization, and the thus obtained acellular matrix is sealed by spraying with a polymer in the form of type II collagen, with a polymer mass concentration of 0. 5%, in 0.01 M hydrochloric acid, and dried. Then, the BAM composite thus obtained is cross-linked by spraying with a cross-linking solution - dialdehyde starch at a mass concentration of 0.1% and in PBS 10x, heated, placed in a Petri dish and PBS solution added until the BAM composite is completely covered and incubated, then frozen again, dried and sterilized with ethylene oxide, and the matrix thus obtained is punched to a given shape, the same as in Example 1.

In another embodiment, the cross-linking solution may also be dialdehyde chitosan or dialdehyde hyaluronate or another factor in the form of glutaraldehyde or aliphatic and aromatic diamine compounds, preferably genipin or EDC/NHS or citric acid or sodium salt of citric acid or tannic acid or pentasodium triphosphate or polyphenol extracts preferably an extract of wine or bitter orange or cranberry peel or green tea.

In other embodiments, the polymers may be type III or IV collagen or cellulose or collagen with cellulose in a ratio of 0.1:2, preferably 1:1 or gelatin or starch or natural silk or cellulose esters and ethers preferably carboxymethyl cellulose or carboxyethyl cellulose or methyl cellulose, or chitosan esters and ethers preferably N or O-carboxymethyl chitosan or O-methyl carboxy chitosan or hyaluronic acid or fibrinogen or a synthetic polymer soluble in organic solvents preferably poly(lactide) or poly(glycolide) or poly(caprolactone) or polyamide or polyimide or poly(carbonate) trimethylene) or poly(dioxanone) or poly(vinyl acetate) or poly(vinyl alcohol) or poly(N-vinyl-2-pyrrolidone) or polyurethane or poly(etherurethane) or polycarbonate or aromatic polycarbonate or poly(ethylene oxide) or polyethylene (ethylene glycol) or poly(acrylic acid) or poly(acrylamide) or poly(acrylonitrile) or poly(vinyl chloride) or a mixture thereof in a ratio of 0.1:2 to 2:1 or a copolymer of preferably L-lactide and caprolactone or poly(L-lactide-co-caprolactone).

### Example XV.

The method of obtaining an implant for the reconstruction of the urinary bladder is that the bladder obtained from a human being is rinsed with a lavage solution and subjected to decellularization, and the thus obtained acellular matrix is sealed by coating with a polymer in the form of type III collagen, at a mass concentration of 2%, in hydrochloric acid with a molar concentration of 0.05 M, and dried, and then the BAM composite thus obtained is crosslinked by coating with a crosslinking solution, in the embodiment in the form of dialdehyde chitosan with a mass concentration of 5%, and in PBS 5x and heated. After heating, a solution of sodium borohydride, preferably 0.1 to 2%, in the embodiment 1%, dissolved in ethanol with a concentration of 90 to 99%, in the embodiment 96%, is applied to the surface of the BAM composite, and left to stabilize during from 1 to 5 hours in an embodiment for 3 hours at a temperature of 0 to 10°C, in an embodiment 4°C. A 0.1 M glycine solution is applied to the surface of the BAM composite and then rinsed with PBS solution.

Then, it is placed in a Petri dish and PBS solution is added until the BAM composite is completely covered and incubated, then re-frozen, dried and sterilized with ethylene oxide, and the resulting matrix is punched to the desired shape. The shape is the same as in example II.

### Example XVI.

Example XVI differs from XV in that after cutting a given shape onto the matrix, previously cultured mesenchymal stromal cells isolated from subcutaneous fat are seeded from the outer part, in a density of 1x10⁶ to 100x10⁶ in the embodiment 10 x 10⁶ cells/cm² by puncturing implant preferably to a depth of 0.1mm to 5mm in an embodiment to 2mm and injection of cells followed by placing the matrix in a bioreactor and incubation for 1 to 30 days in an embodiment 7 days in an atmosphere of 2 to 10% in an embodiment of 5% CO2, and a temperature of 22 to 40°C in an embodiment of 37°C.

In terms of cross-linking, in other embodiments, mesenchymal stromal cells can also be isolated from bone marrow or Wharton's jelly or other vascularized human tissues. The cell density will be from 1x10⁶ to 100x10⁶ at each point.

In other embodiments, the polymers may be type III or IV collagen or cellulose or collagen with cellulose in a ratio of 0.1:2, preferably 1:1 or gelatin or starch or natural silk or cellulose esters and ethers preferably carboxymethyl cellulose or carboxyethyl cellulose or methyl cellulose, or chitosan esters and ethers preferably N or O-carboxymethyl chitosan or O-methyl carboxy chitosan or hyaluronic acid or fibrinogen or a synthetic polymer soluble in organic solvents preferably poly(lactide) or poly(glycolide) or poly(caprolactone) or polyamide or polyimide or poly(carbonate) trimethylene) or poly(dioxanone) or poly(vinyl acetate) or poly(vinyl alcohol) or poly(N-vinyl-2-pyrrolidone) or polyurethane or poly(etherurethane) or polycarbonate or aromatic polycarbonate or poly(ethylene oxide) or polyethylene (ethylene glycol) or poly(acrylic acid) or poly(acrylamide) or poly(acrylonitrile) or poly(vinyl chloride) or a mixture thereof in a ratio of 0.1:2 to 2:1 or a copolymer of preferably L-lactide and caprolactone or poly(L-lactide-co-caprolactone).

### Example XVII

Example XVII differs from XV in that the cut implant has an axisymmetric shape, a three-armed implant with arms resembling a lily petal inscribed in a circle with an area of 3 cm2, described inside a diameter D of 2.99 cm and a thickness of 0.2 cm, with an opening angle of alpha 8 °, radius R1 4.52 mm and radius R2 2.23 mm, and point angle beta 24.6°.

## Claims

1. An implant for the reconstruction of the urinary bladder, **characterized in that** it is in the form of a multi-arm, preferably three-arm, axisymmetric element with arms inscribed in a circle and made of acellular matrix cross-linked with a polymer, the acellular matrix being made of decellularized tissue or an organ of a mammal, preferably human or pig most preferably from the bladder or intestinal wall or skin or dura or pericardium or fragments thereof.

2. The implant according to claim 1, **characterized in that** it has a shape with arms resembling a lily petal inscribed in a circle with an area of 3 cm2 to 60 cm2, preferably 30 cm2, described inside a diameter D of 2.9 cm to 14 cm, preferably 9, 45 cm and a thickness of 0.1 cm to 1 cm, preferably 0.5 cm, with an angle of opening alpha from 8° to 37°, preferably 26°, radius R1 from 4.5 mm to 20.5 mm, preferably 14.3 mm and radius R2 from 2.2 mm to 10.02 mm preferably 7.08 mm, and apex angle beta from 24° to 110° preferably 77.7°, preferably the graft size of any surface (S) for D, R1 and R2 multiplies times the scale factor (x) based on the formula: $x = \sqrt{\frac{S}{30}}$

3. Implant according to claim 1 or 2, **characterized in that** the polymer is type I or II or III or IV collagen, preferably of animal origin, or cellulose or collagen with cellulose in a ratio of 0.1:2, preferably 1:1, or gelatin or starch or natural silk or cellulose esters and ethers preferably carboxymethyl cellulose or carboxyethyl cellulose or methyl cellulose or chitosan esters and ethers preferably N or O-carboxymethyl chitosan or O-methyl carboxy chitosan or hyaluronic acid or fibrinogen or a synthetic polymer soluble in organic solvents preferably poly(lactide) or poly (glycolide) or poly(caprolactone) or polyamide or polyimide or poly(trimethylene carbonate) or poly(dioxanone) or poly(vinyl acetate) or poly(vinyl alcohol) or poly(N-vinyl-2-pyrrolidone) or polyurethane or poly (etherurethane) or polycarbonate or aromatic polycarbonate or polyethylene oxide or polyethylene glycol or polyacrylic acid or polyacrylamide or polyacrylonitrile or poly(vinyl chloride) or a mixture thereof in a ratio of 0.1:2 to 2:1 or a copolymer of preferably L-lactide and caprolactone or poly(L-lactide-co-caprolactone).

4. The implant according to claim 1, 2 or 3, **characterized in that** the polymer-crosslinked cell-free matrix comprises on the surface or inside mesenchymal stromal cells isolated from bone marrow, subcutaneous fat, Wharton's jelly or other vascularized human tissues, preferably in a density of 1x10⁶ to 100x10⁶ preferably 10x10⁶ cells/cm².

5. A method of obtaining an implant for the reconstruction of the bladder, the obtained tissue or organ of a mammal, preferably human or porcine, preferably from the bladder or intestinal wall or skin or dura or pericardium or fragments thereof, is rinsed with a washing solution and subjected to decellularization, and the cell-free matrix thus obtained is sealed by dipping, spraying, padding or coating with a polymer with a polymer mass concentration of 0.5 to 2%, preferably 1% in hydrochloric acid with a molar concentration of 0.01M to 0.05M, preferably 0.17M and dried, and then the BAM composite thus obtained is crosslinked by dipping, spraying, padding or coating with a crosslinking solution, preferably a dialdehyde natural polymer with a mass concentration of 0.1 to 5%, preferably 1% and in PBS from 10x to 1x, preferably 1x , heats, puts in a Petri dish and adds PBS solution until the BAM composite is completely covered and incubated, then it is re-frozen, dried and sterilized with ethylene oxide, and the thus obtained matrix is punched to a given shape, where the polymer is type I or II or III or IV collagen, preferably of animal origin, or cellulose or collagen to cellulose in a ratio of 0.1:2, preferably 1:1, or gelatin or starch or natural silk or cellulose esters and ethers preferably carboxymethylcellulose or carboxyethylcellulose or methylcellulose or chitosan esters and ethers preferably N or O-carboxymethylchitosan or O-methylcarboxychitosan or hyaluronic acid or fibrinogen or a synthetic polymer soluble in organic solvents preferably poly(lactide) or poly(glycolide) or poly(caprolactone) or polyamide or polyimide or poly(trimethylene carbonate) or poly(dioxanone) or poly(vinyl acetate) or poly(vinyl alcohol) or poly(N-vinyl-2-pyrrolidone) or polyurethane or poly(etherurethane) or polycarbonate or aromatic polycarbonate or poly(ethylene oxide) or poly(ethylene glycol) or poly(acrylic acid) or poly(acrylamide) or poly(acrylonitrile) or poly(vinyl chloride) or a mixture thereof in a ratio of 0.1:2 to 2:1 or a copolymer of preferably L-lactide and caprolactone or poly(L-lactide-co-caprolactone), and after heating, a solution of sodium borohydride, preferably 0.1 to 2%, most preferably 1%, dissolved in ethanol with a concentration of 90 to 99%, preferably 96%, is applied to the surface of the BAM composite , and allowed to stabilize, preferably for 1 to 5 hours, most preferably for 3 hours, preferably at a temperature of 0 to 10°C, most preferably 4°C, preferably a 0.1 M glycine solution is applied to the surface of the BAM composite and then rinsed with a PBS solution, and before or after cutting a predetermined shape onto a matrix from the outer part, previously cultured stromal mesenchymal cells isolated from bone marrow, subcutaneous fat, Wharton's jelly or other vascularized human tissues are seeded in density from 1x10⁶ to 100x10⁶, preferably 10 x 10⁶ cells/cm² by puncturing the implant preferably to a depth of from 0.1 mm to 5 mm, preferably to 2 mm, and injecting the cells, or by depositing the cells on the surface of the implant, and then placing the matrix in the bioreactor and incubates, most preferably for 1 to 30 days, preferably 7 days, in an atmosphere of 2 to 10%, preferably 5% CO₂, and a temperature of 22 to 40°C, preferably 37°C **characterized in that** the matrix has a shape in the form of a multi-arm, preferably three-arm, axisymmetric element with arms inscribed in a circle and made of a cell-free matrix cross-linked with a polymer, the cell-free matrix being made of from a decellularized tissue or organ of a mammal, preferably human or porcine, most preferably from the bladder or intestinal wall or skin or dura or pericardium or fragments thereof.

6. Method according to claims 5 **characterized in that** the scaffold has a shape with arms resembling a lily petal inscribed in a circle with an area of 3 cm2 to 60 cm2, preferably 30 cm2, described inside a diameter D of 2.9 cm to 14 cm, preferably 9.45 cm, and a thickness of 0.1 cm to 1 cm, preferably 0.5 cm, with a flare angle alpha from 8° to 37°, preferably 26°, radius R1 from 4.5 mm to 20 .5 mm, preferably 14.3 mm, and the radius R2 from 2.2 mm to 10.02 mm, preferably 7.08 mm, and the apex angle beta from 24° to 110°, preferably 77.7°, preferably when the size of the implant of any surface (S) for D, R1 and R2, multiply the scale factor (x) by the formula: $x = \sqrt{\frac{S}{30}}$

## Patentansprüche

1. Implantat zur Rekonstruktion der Harnblase, **dadurch gekennzeichnet, dass** es als mehrarmiges, vorzugsweise dreiarmiges, achsensymmetrisches Element ausgebildet ist, dessen Arme in einen Kreis eingeschrieben sind, und aus einer mit einem Polymer vernetzten azellulären Matrix hergestellt ist, wobei die azelluläre Matrix aus einem dezellularisierten Gewebe oder Organ eines Säugetiers, vorzugsweise eines Menschen oder eines Schweins, besonders bevorzugt aus der Harnblase, der Darmwand, der Haut, der Dura mater oder dem Perikard bzw. Fragmenten derselben hergestellt ist.

2. Implantat nach Anspruch 1, **dadurch gekennzeichnet, dass** es die Form eines Elements mit lilienblütenblattförmigen Armen aufweist, die in einen Kreis mit einer Fläche von 3 cm² bis 60 cm², vorzugsweise 30 cm², eingeschrieben sind, wobei der Kreis durch einen Durchmesser D von 2,9 cm bis 14 cm, vorzugsweise 9,45 cm, beschrieben ist, und das Implantat eine Dicke von 0,1 cm bis 1 cm, vorzugsweise 0,5 cm, einen Öffnungswinkel α von 8° bis 37°, vorzugsweise 26°, einen Radius R1 von 4,5 mm bis 20,5 mm, vorzugsweise 14,3 mm, einen Radius R2 von 2,2 mm bis 10,02 mm, vorzugsweise 7,08 mm, sowie einen Scheitelwinkel β von 24° bis 110°, vorzugsweise 77,7°, aufweist, wobei vorzugsweise für ein Implantat mit einer beliebigen Fläche (S) die Werte D, R1 und R2 mittels Multiplikation mit dem Skalierungsfaktor (x) nach folgender Formel bestimmt werden: $x = \sqrt{\frac{S}{30}}$

3. Implantat nach Anspruch 1 oder 2, **dadurch gekennzeichnet, dass** das Polymer Kollagen des Typs I, II, III oder IV, vorzugsweise tierischen Ursprungs, oder Cellulose oder Kollagen mit Cellulose in einem Verhältnis von 0,1:2, vorzugsweise 1:1, oder Gelatine oder Stärke oder Naturseide oder Celluloseester und -ether, vorzugsweise Carboxymethylcellulose oder Carboxyethylcellulose oder Methylcellulose, oder Chitosanester und -ether, vorzugsweise N- oder O-Carboxymethylchitosan oder O-Methylcarboxychitosan, oder Hyaluronsäure oder Fibrinogen oder ein in organischen Lösungsmitteln lösliches synthetisches Polymer, vorzugsweise Poly(lactid) oder Poly(glycolid) oder Poly(caprolacton) oder Polyamid oder Polyimid oder Poly(trimethylencarbonat) oder Poly(dioxanon) oder Poly(vinylacetat) oder Poly(vinylalkohol) oder Poly(N-vinyl-2-pyrrolidon) oder Polyurethan oder Poly(etherurethan) oder Polycarbonat oder aromatisches Polycarbonat oder Poly(ethylenoxid) oder Poly(ethylenglycol) oder Poly(acrylsäure) oder Poly(acrylamid) oder Poly(acrylnitril) oder Poly(vinylchlorid) oder eine Mischung derselben in einem Verhältnis von 0,1:2 bis 2:1 oder ein Copolymer, vorzugsweise aus L-Lactid und Caprolacton, oder Poly(L-lactid-co-caprolacton) ist.

4. Implantat nach Anspruch 1, 2 oder 3, **dadurch gekennzeichnet, dass** die mit einem Polymer vernetzte azelluläre Matrix auf ihrer Oberfläche oder in ihrem Inneren mesenchymale Stromazellen enthält, die aus dem Knochenmark, dem subkutanen Fettgewebe, der Wharton-Sulze oder anderen vaskularisierten Geweben des Menschen isoliert wurden, vorzugsweise in einer Zelldichte von 1 × 10⁶ bis 100 × 10⁶, vorzugsweise 10 × 10⁶ Zellen/cm².

5. Verfahren zur Herstellung eines Implantats zur Rekonstruktion der Harnblase, bei dem ein entnommenes Gewebe oder Organ eines Säugetiers, vorzugsweise eines Menschen oder eines Schweins, vorzugsweise aus der Harnblase, der Darmwand, der Haut, der Dura mater oder dem Perikard bzw. Fragmenten derselben, mit einer Spüllösung gespült und einer Dezellularisierung unterzogen wird, wobei die auf diese Weise erhaltene azelluläre Matrix durch Eintauchen, Besprühen, Auftragsschweißen oder Beschichten mit einer Polymerlösung mit einer Polymerkonzentration von 0,5 bis 2 Gew.-%, vorzugsweise 1 Gew.-%, in Salzsäure mit einer Molarität von 0,01 M bis 0,05 M, vorzugsweise 0,17 M, beschichtet und anschließend getrocknet wird, woraufhin der auf diese Weise erhaltene BAM-Verbundwerkstoff durch Eintauchen, Besprühen, Auftragsschweißen oder Beschichten mit einer Vernetzungslösung, die vorzugsweise ein dialdehydhaltiges natürliches Polymer mit einer Konzentration von 0,1 bis 5 Gew.-%, vorzugsweise 1 Gew.-%, in PBS von 10× bis 1×, vorzugsweise 1×, enthält, vernetzt, anschließend erwärmt, auf eine Petrischale gelegt, mit PBS-Lösung bis zur vollständigen Bedeckung des BAM-Verbundwerkstoffs versetzt und inkubiert sowie anschließend erneut eingefroren, getrocknet und mit Ethylenoxid sterilisiert wird, woraufhin die auf diese Weise erhaltene Matrix in die vorgegebene Form ausgestanzt wird, wobei hierfür als Polymer Kollagen des Typs I, II, III oder IV, vorzugsweise tierischen Ursprungs, oder Cellulose oder Kollagen mit Cellulose in einem Verhältnis von 0,1:2, vorzugsweise 1:1, oder Gelatine oder Stärke oder Naturseide oder Celluloseester und -ether, vorzugsweise Carboxymethylcellulose oder Carboxyethylcellulose oder Methylcellulose, oder Chitosanester und -ether, vorzugsweise N- oder O-Carboxymethylchitosan oder O-Methylcarboxychitosan, oder Hyaluronsäure oder Fibrinogen oder ein in organischen Lösungsmitteln lösliches synthetisches Polymer, vorzugsweise Poly(lactid) oder Poly(glycolid) oder Poly(caprolacton) oder Polyamid oder Polyimid oder Poly(trimethylencarbonat) oder Poly(dioxanon) oder Poly(vinylacetat) oder Poly(vinylalkohol) oder Poly(N-vinyl-2-pyrrolidon) oder Polyurethan oder Poly(etherurethan) oder Polycarbonat oder aromatisches Polycarbonat oder Poly(ethylenoxid) oder Poly(ethylenglycol) oder Poly(acrylsäure) oder Poly(acrylamid) oder Poly(acrylnitril) oder Poly(vinylchlorid) oder eine Mischung derselben in einem Verhältnis von 0,1:2 bis 2:1 oder ein Copolymer, vorzugsweise aus L-Lactid und Caprolacton, oder Poly(L-lactid-co-caprolacton) verwendet wird, wobei nach dem Erwärmen auf die Oberfläche des BAM-Verbundwerkstoffs eine in Ethanol mit einer Konzentration von 90 bis 99 %, vorzugsweise 96 %, gelöste Natriumborhydridlösung mit einer Konzentration von 0,1 bis 2 %, besonders bevorzugt 1 %, aufgebracht und zur Stabilisierung stehen gelassen wird, vorzugsweise für eine Dauer von 1 bis 5 Stunden, besonders bevorzugt 3 Stunden, vorzugsweise bei einer Temperatur von 0 bis 10 °C, besonders bevorzugt 4 °C, wobei vorzugsweise anschließend eine 0,1-M-Glycinlösung auf die Oberfläche des BAM-Verbundwerkstoffs aufgebracht und danach mit PBS-Lösung gespült wird, wobei vor oder nach dem Ausstanzen der vorgegebenen Form auf die Außenseite der Matrix zuvor kultivierte mesenchymale Stromazellen, die aus dem Knochenmark, dem subkutanen Fettgewebe, der Wharton-Sulze oder anderen vaskularisierten Geweben des Menschen isoliert wurden, in einer Zelldichte von 1 × 10⁶ bis 100 × 10⁶, vorzugsweise 10 × 10⁶ Zellen/cm², durch Einstechen in das Implantat, vorzugsweise bis zu einer Tiefe von 0,1 mm bis 5 mm, vorzugsweise bis zu 2 mm, und Injektion der Zellen oder durch Aufbringen der Zellen auf die Oberfläche des Implantats ausgesät werden, woraufhin die Matrix in einem Bioreaktor inkubiert wird, besonders bevorzugt für eine Dauer von 1 bis 30 Tagen, vorzugsweise 7 Tagen, in einer Atmosphäre mit 2 bis 10 %, vorzugsweise 5 % CO₂, bei einer Temperatur von 22 bis 40 °C, vorzugsweise 37 °C, **dadurch gekennzeichnet, dass** die Matrix die Form eines mehrarmigen, vorzugsweise dreiarmigen, achsensymmetrischen Elements mit in einen Kreis eingeschriebenen Armen aufweist und aus einer mit einem Polymer vernetzten azellulären Matrix besteht, wobei die azelluläre Matrix aus einem dezellularisierten Gewebe oder Organ eines Säugetiers, vorzugsweise eines Menschen oder eines Schweins, besonders bevorzugt aus der Harnblase, der Darmwand, der Haut, der Dura mater oder dem Perikard bzw. Fragmenten derselben besteht.

6. Verfahren nach Anspruch 5, **dadurch gekennzeichnet, dass** das Gerüst die Form eines Elements mit lilienblattförmigen, in einen Kreis eingeschriebenen Armen aufweist, wobei der Kreis eine Fläche von 3 cm² bis 60 cm², vorzugsweise 30 cm², umfasst und durch einen Durchmesser D von 2,9 cm bis 14 cm, vorzugsweise 9,45 cm, beschrieben ist, wobei das Gerüst ferner eine Dicke von 0,1 cm bis 1 cm, vorzugsweise 0,5 cm, einen Öffnungswinkel α von 8° bis 37°, vorzugsweise 26°, einen Radius R1 von 4,5 mm bis 20,5 mm, vorzugsweise 14,3 mm, einen Radius R2 von 2,2 mm bis 10,02 mm, vorzugsweise 7,08 mm, sowie einen Scheitelwinkel β von 24° bis 110°, vorzugsweise 77,7°, aufweist, wobei vorzugsweise für ein Implantat mit einer beliebigen Fläche (S) die Werte D, R1 und R2 mit dem Skalierungsfaktor (x) gemäß der nachstehenden Formel multipliziert werden: $x = \sqrt{\frac{S}{30}}$

## Revendications

1. Implant destiné à la reconstruction de la vessie, **caractérisé en ce qu'**il se présente sous la forme d'un élément à plusieurs branches, de préférence à trois branches, à symétrie axiale, dont les branches sont inscrites dans un cercle, et constitué d'une matrice acellulaire réticulée par un polymère, la matrice acellulaire étant constituée de tissu ou d'organe de mammifère décellularisé, de préférence humain ou porcin, et plus particulièrement provenant de la vessie, de la paroi intestinale, de la peau, de la dure-mère ou du péricarde, ou de fragments de ceux-ci.

2. Implant selon la revendication 1, **caractérisé en ce qu'**il présente une forme dont les branches rappellent un pétale de lys inscrit dans un cercle d'une surface comprise entre 3 cm² et 60 cm², de préférence 30 cm², dont le diamètre intérieur D est compris entre 2,9 cm et 14 cm, de préférence 9,45 cm, et d'une épaisseur comprise entre 0,1 cm et 1 cm, de préférence 0,5 cm, avec un angle d'ouverture alpha compris entre 8° et 37°, de préférence 26°, d'un rayon R1 compris entre 4,5 mm et 20,5 mm, de préférence 14,3 mm, et d'un rayon R2 compris entre 2,2 mm et 10,02 mm, de préférence 7,08 mm, et d'un angle au sommet bêta compris entre 24° et 110°, de préférence 77,7° ; de préférence, la taille de l'implant de n'importe quelle surface (S) pour D, R1 et R2 est multipliée par un facteur d'échelle (x) selon la formule suivante : $x = \sqrt{\frac{S}{30}}$

3. Implant selon la revendication 1 ou 2, **caractérisé en ce que** le polymère est du collagène de type I, Il, III ou IV, de préférence d'origine animale, ou de la cellulose, ou du collagène avec de la cellulose dans un rapport de 0,1:2, de préférence 1:1, ou de la gélatine, ou de l'amidon, ou de la soie naturelle, ou des esters et éthers de cellulose, de préférence la carboxyméthylcellulose, la carboxyéthylcellulose, la méthylcellulose, ou des esters et éthers de chitosane, de préférence N ou O -carboxyméthylchitosane, ou O-méthylcarboxychitosane, ou acide hyaluronique, ou fibrinogène, ou polymère synthétique soluble dans les solvants organiques, de préférence le poly(lactide), le poly(glycolide), le poly(caprolactone), le polyamide, le polyimide, le poly(carbonate de triméthylène), le poly (dioxanone), ou le poly(acétate de vinyle), ou le poly(alcool vinylique), ou le poly(N-vinyl-2-pyrrolidone), ou le polyuréthane, ou le poly(étheruréthane), ou le polycarbonate, ou le polycarbonate aromatique, ou le poly(oxyde d'éthylène), ou le poly (éthylène glycol) ou le poly(acide acrylique) ou le poly(acrylamide) ou le poly(acrylonitrile) ou le poly(chlorure de vinyle) ou d'un mélange de ceux-ci dans un rapport compris entre 0,1:2 et 2:1, ou d'un copolymère, de préférence de L-lactide et de caprolactone, ou de poly(L-lactide-co-caprolactone).

4. Implant selon la revendication 1, 2 ou 3, **caractérisé en ce que** la matrice acellulaire réticulée par un polymère contient, à sa surface ou à l'intérieur, des cellules mésenchymateuses stromales isolées à partir de la moelle osseuse, du tissu adipeux sous-cutané, de la gelée de Wharton ou d'autres tissus vasculaires humains, de préférence à une densité comprise entre 1x10⁶ et 100x10⁶, de préférence 10x10⁶ cellules/cm².

5. Procédé d'obtention d'un implant destiné à la reconstruction de la vessie, dans lequel le tissu ou l'organe prélevé sur un mammifère, de préférence un être humain ou un porc, provenant de préférence de la vessie, de la paroi intestinale, de la peau, de la dure-mère ou du péricarde, ou de fragments de ceux-ci, est rincé avec une solution de rinçage et soumis à une décellularisation, et la matrice acellulaire ainsi obtenue est soumise à une étanchéification par immersion, pulvérisation, par projection ou par enrobage avec un polymère dont la concentration est comprise entre 0,5 et 2 % en poids, de préférence 1 % en poids, dans de l'acide chlorhydrique dont la concentration molaire est comprise entre 0,01 M et 0,05 M, de préférence 0,17 M, puis séchée ; ensuite, le composite BAM ainsi obtenu est réticulé par immersion, pulvérisation, projection ou enduction avec une solution de réticulation, de préférence un polymère naturel dialdéhyde à une concentration comprise entre 0,1 et 5 % en poids, de préférence 1 % en poids, et dans du PBS à une dilution de 10x à 1x, de préférence 1x, on le chauffe, on le place dans une boîte de Pétri et on ajoute une solution de PBS jusqu'à recouvrir entièrement le composite BAM, puis on l'incube ; ensuite, il es congelé à nouveau, séché et stérilisé à l'oxyde d'éthylène, et la matrice ainsi obtenue est découpée à la forme souhaitée, le polymère étant du collagène de type I, II, III ou IV, de préférence d'origine animale, ou de la cellulose, ou encore du collagène associé à de la cellulose dans un rapport de 0,1:2, de préférence 1:1, ou de la gélatine, ou de l'amidon, ou de la soie naturelle, ou des esters et éthers de cellulose, de préférence la carboxyméthylcellulose, la carboxyéthylcellulose, la méthylcellulose, ou des esters et éthers de chitosane, de préférence N ou O-carboxyméthylchitosane, ou O-méthylcarboxychitosane, ou acide hyaluronique, ou fibrinogène, ou polymère synthétique soluble dans les solvants organiques, de préférence le poly(lactide) ou le poly(glycolide), ou le poly(caprolactone), ou le polyamide, ou le polyimide, ou le poly(carbonate de triméthyle), ou le poly(dioxanone), ou le poly(octane de vinyle), ou le poly(alcool vinylique), ou le poly(N-vinyl-2-pyrrolidone), ou du polyuréthane, ou du poly(étheruréthane), ou du polycarbonate, ou du polycarbonate aromatique, ou du poly(oxyde d'éthylène), ou du poly(éthylène glycol), ou du poly(acide acrylique), ou du poly(acrylamide), ou du poly(acrylonitrile) ou le poly(chlorure de vinyle) ou un mélange de ceux-ci dans un rapport compris entre 0,1:2 et 2:1 ou un copolymère, de préférence de L-lactide et de caprolactone, ou le poly(L-lactide-co-caprolactone), et, après chauffage, on applique sur la surface du composite BAM une solution de borohydrure de sodium, de préférence de 0,1 à 2 %, de préférence 1 %, dissoute dans de l'éthanol à une concentration comprise entre 90 et 99 %, de préférence 96 %, et on laisse stabiliser, de préférence pendant 1 à 5 heures, de préférence pendant 3 heures, de préférence à une température comprise entre 0 et 10 °C, de préférence à 4 °C ; de préférence, on applique sur la surface du composite BAM une solution de 0,1 M de glycine, puis on rince avec une solution de PBS, et avant ou après avoir découpé la forme souhaitée à l'aide d'une matrice, on ensemence depuis la face externe des cellules mésenchymateuses stromales préalablement cultivées, isolées de la moelle osseuse, du tissu adipeux sous-cutané, de la gelée de Wharton ou d'autres tissus vascularisés humains, à une densité comprise entre 1 × 10⁶ et 100 × 10⁶, de préférence10 × 10⁶cellules/cm², soit par ponction de l'implant, de préférence à une profondeur comprise entre 0,1 mm et 5 mm, de préférence jusqu'à 2 mm, et injection des cellules, soit par dépôt des cellules à la surface de l'implant, puis on place la matrice dans un bioréacteur et on l'incube, de préférence pendant une durée comprise entre 1 et 30 jours, de préférence 7 jours, dans une atmosphère contenant entre 2 et 10 %, de préférence 5 % de CO₂, et à une température comprise entre 22 et 40 °C, de préférence à 37 °C, **caractérisée en ce que** la matrice a la forme d'un élément à plusieurs branches, de préférence à trois branches, à symétrie axiale, dont les branches sont inscrites dans un cercle, et est constituée d'une matrice acellulaire réticulée par un polymère, la matrice sans alvéoles étant constituée de tissu ou d'organe de mammifère décellularisé, de préférence humain ou porcin, et de manière particulièrement préférée provenant de la vessie, de la paroi intestinale, de la peau, de la dure-mère, du péricarde ou de fragments de ceux-ci.

6. Procédé selon la revendication 5, **caractérisé en ce que** la structure présente une forme dont les branches rappellent un pétale de lys inscrit dans un cercle d'une surface comprise entre 3 cm² et 60 cm², de préférence 30 cm², dont le diamètre intérieur D est compris entre 2,9 cm et 14 cm, de préférence 9,45 cm, et d'une épaisseur comprise entre 0,1 cm et 1 cm, de préférence 0,5 cm, avec un angle d'ouverture alpha compris entre 8° et 37°, de préférence 26°, d'un rayon R1 compris entre 4,5 mm et 20,5 mm, de préférence 14,3 mm, et d'un rayon R2 compris entre 2,2 mm et 10,02 mm, de préférence 7,08 mm, et d'un angle au sommet bêta compris entre 24° et 110°, de préférence 77,7° ; de préférence, la taille de l'implant de n'importe quelle surface (S) pour D, R1 et R2 est multipliée par un facteur d'échelle (x) selon la formule suivante : $x = \sqrt{\frac{S}{30}}$
